# FASCICULE DE BREVET EUROPEEN

(11) **EP 2 317 974 B1**
(45) Date de publication et mention de la délivrance du brevet: **25.03.2015**
(21) Numéro de dépôt: 09784295.9
(22) Date de dépôt: 23.07.2009
(51) Int. Cl.: A23K 1/18, A61K 9/28, A61K 9/48, A61K 31/197, A61K 31/4164, A23K 1/00, A61K 9/00

(54) **MEDICAMENTS APPETISSANTS A ADMINISTRATION ORALE SOUS FORME SOLIDE POUR LA PREVENTION ET/OU LE TRAITEMENT DE L'INSUFFISANCE CARDIAQUE CHEZ LES ANIMAUX**
APPETITANREGENDES MEDIKAMENT ZUR ORALEN VERABREICHUNG IN FESTER FORM ZUR PRÄVENTION UND/ODER BEHANDLUNG VON HERZINSUFFIZIENZ BEI TIEREN
APPETISING MEDICAMENTS FOR ORAL ADMINISTRATION IN SOLID FORM FOR PREVENTION AND/OR TREATMENT OF CARDIAC INSUFFICIENCY IN ANIMALS

(30) Priorité: 23.07.2008 FR 0804184
(43) Date de publication de la demande: 11.05.2011
(73) Titulaire: VIRBAC, 06516 Carros (FR)
(72) Inventeur: DERRIEU, Guy, F-06800 Cagnes Sur Mer (FR); CORVAISIER, David, F-06560 Valbonne (FR)
(74) Mandataire: Bernstein, Claire Jacqueline
(86) Numéro de dépôt international: PCT/FR2009/000917
(87) Numéro de publication internationale: WO 2010/010257

(56) Documents cités:
- EP-A- 0 025 226
- EP-A- 0 320 320
- WO-A-89/12442
- DE-A1- 4 001 622
- FR-A- 2 350 105
- US-A- 5 683 722

## Description

La présente invention se rapporte au domaine de la préparation de médicaments vétérinaires et vise à améliorer la prise orale d'une composition pharmaceutique vétérinaire solide à administration orale pour la prévention et/ou le traitement de l'insuffisance cardiaque chez les animaux. La présente invention vise plus particulièrement à améliorer la prise orale du pimobendan, 4,5dihydro-6-[2-(4-methoxyphenyl)-1 H-benzimidazol-5-yl]-5-methyl-3(2H)-pyridazinone, formulé sous une forme solide, par les carnivores.

Le pimobendan, décrit dans le brevet européen EP 0 008 391, est une substance possédant des activités cardiotoniques, hypotensives, et anti-thrombotiques. C'est la substance de référence dans le traitement de l'insuffisance cardiaque.

La présente invention est une composition pharmaceutique vétérinaire solide appétissante à administration orale pour la prévention et/ou le traitement de l'insuffisance cardiaque chez les animaux, facilitant la prise orale spontanée et l'administration de substances médicamenteuses par des carnivores domestiques et en particulier les chiens et les chats. On assiste actuellement à une évolution des soins qui sont prodigués aux animaux de compagnie, et la voie orale devient une voie privilégiée pour l'administration de médicaments par le professionnel de santé ou par le propriétaire.

En effet, les voies d'administration parentérales usuelles (intramusculaire, sous-cutanée, intradermique ou intraveineuse) des médicaments présentent certains inconvénients. Les voies intramusculaire ou sous-cutanée peuvent, par exemple, être à l'origine d'hématomes ou d'abcès. Quant à la voie intraveineuse, elle requiert souvent l'intervention d'un spécialiste (vétérinaire). Ces voies d'administration parentérales nécessitent également la contention des animaux. Par ailleurs, certains principes actifs sont difficiles à formuler dans des formes galéniques parentérales. Enfin, certains principes actifs n'exerceront leur action thérapeutique chez l'animal que s'ils arrivent directement dans l'appareil digestif.

Les formulations galéniques adaptées à l'administration de médicaments par voie orale ou *per os* se présentent généralement sous forme liquide (tels que des sirops, des solutions ou des suspensions buvables, des gouttes...), sous forme semi-solide (telle que les pâtes pour administration orale) ou sous forme solide. Les formes solides couramment utilisées pour administrer des médicaments aux animaux de compagnie, en particulier les chats et les chiens, se présentent sous diverses formulations de natures différentes et sont obtenues par différents procédés. On distingue par exemple les gélules dures, les capsules molles, les cachets, les capsules, les comprimés, les gommes à mâcher, les pilules, les pastilles, les tablettes.

La forme gélule dure est particulièrement préférée. Sous cette forme la substance active se trouve dans un environnement particulièrement favorable à sa stabilité. On peut ainsi en effet limiter toutes sortes de dégradation dues au contact avec d'autres substances telles que les excipients, charges, matières appétissantes, qui surviennent lors de l'élaboration d'autres formes solides pour administration orale telle que les comprimés, pilules, pastilles, tablettes dont la fabrication met en oeuvre des contraintes physiques et mécaniques importantes.

On constate que l'observance des traitements administrés par voie orale (c'est-à-dire le respect des consignes et directives du professionnel de santé concernant la prise des médicaments) n'est pas toujours convenablement suivie, en raison de la difficulté à administrer en totalité les traitements aux animaux. En effet, l'administration aux animaux de médicaments sous forme galénique solide par voie orale est souvent difficile du fait du mauvais goût de certaines substances actives ou de certains excipients constituant le médicament et du sens très développé de l'odorat et du goût des animaux. De plus, l'administration orale d'un médicament implique, dans la majorité des cas, l'utilisation de doses variables en fonction des individus et des pathologies considérées et nécessite parfois d'être fractionnée et répétée dans le temps. On a observé, particulièrement chez les chiens et chats, que la principale raison qui rend très difficile, voire impossible, l'observance d'un traitement oral est le défaut d'appétence suscitée par le médicament.

L'appétence pour un médicament se définit comme l'état psychologique correspondant à un désir d'absorber le médicament en réponse à la perception des caractères organoleptiques de celui-ci. La capacité à susciter l'appétence est appelée appétissance. Elle correspond à la combinaison des caractères d'un médicament qui déterminent l'attrait qu'il aura sur des animaux normalement nourris. L'appétissance d'un médicament participe grandement au refus ou à l'acceptation par l'animal de la prise spontanée du traitement et de la répétition de la prise sur des périodes parfois longues. Dans le cadre de certains traitements, la prise du traitement peut être journalier et à vie.

L'appétissance d'un médicament administré par voie orale conduit à l'acceptation et à l'ingestion volontaire par les animaux. Cette appétissance peut être mesurée dans un essai général d'appétence prenant en compte différents paramètres du médicament, comme sa prise spontanée à la main ou au sol, ou encore sa consommation, même s'il y a plusieurs médicaments à prendre en une prise ou à intervalles réguliers par l'animal.

Dans l'art antérieur, de nombreuses solutions ont été mises au point pour faciliter l'absorption des médicaments par l'animal :

### 1. Une première option consiste à masquer le goût et/ou l'odeur désagréable du ou des principes actifs par encapsulation ou par enrobage de ces derniers.

Ainsi, dans la Demande de Brevet EP 0 997 143, le problème d'amertume des principes actifs est résolu en les encapsulant dans des compositions qui masquent le goût, telles que des mélanges d'acétate de cellulose ou d'acétate butyrate de cellulose et de polyvinylpyrrolidone (PVP) ou d'hydroxypropyl cellulose (HPC).

La Demande de Brevet EP 1 490 037 propose la préparation d'un médicament dont le goût désagréable du principe actif est masqué ; la préparation consiste à enrober le principe actif dont on cherche à masquer le goût autour de granules servant de support ; la couche de principe actif est ensuite recouverte d'une couche protectrice réalisée avec une matrice polymérique physiologiquement acceptable (telle que cellulose, amidon, saccharose, lactose ou d'autres types de sucre) qui évite au principe actif d'être directement en contact avec les cellules gustatives de l'animal. Les granules ainsi préparés peuvent ensuite être mélangés avec une substance appétissante, le mélange est alors comprimé sous forme de tablette par exemple.

Cependant, ces solutions nécessitent de nombreuses étapes d'encapsulation ou d'enrobage. De plus, la préparation des produits médicamenteux sous forme galénique solide impose aux principes actifs d'être directement au contact d'autres substances comme l'eau ou les solvants organiques qui permettent soit d'enrober les principes actifs par des polymères, soit obtenir des principes actifs en solution, solution servant en l'enrobage de supports par ces mêmes principes actifs. La fabrication se poursuit ensuite généralement par compression ; or, certains principes actifs sont très fragiles et ne résistent pas aux contraintes chimiques et physiques mises en oeuvre lors de la préparation des produits médicamenteux avec, comme étape finale, la compression.

La Demande Internationale WO 01/15547 décrit une formulation d'agents actifs mal acceptés par l'animal pour l'utilisation vétérinaire. Là encore, le principe proposé est d'encapsuler le ou les agents actifs, préalablement incorporés dans une matrice, pour en masquer le goût. La matrice intégrant les agents actifs est recouverte d'une couche ayant un goût neutre comprenant du xanthane, du polyvinylpyrrolidone et du lauryl-sulfate de sodium. Il n'y a pas de substance appétissante utilisée. Ainsi formulé(s), le ou les agents actifs ne sont plus perçu(s) par l'animal.

Cependant, le procédé décrit dans ce document nécessite notamment une étape d'extrusion qui est susceptible de dégrader les principes actifs fragiles.

### 2. Selon une autre option, les principes actifs sont mélangés dans une matrice comprenant une substance appétissante afin de masquer leur goût.

Ainsi, la Demande de Brevet australien AU2001279664 décrit un produit extrudé à base d'amidon contenant un principe actif et un arôme spécifique, apte à être administré oralement à un animal.

Cependant, l'extrusion peut poser des problèmes de dégradation de certains principes actifs pour lesquels il est préférable de l'éviter.

Par ailleurs, un arôme est un principe odorant d'origine synthétique ou naturelle qui ne sera perçu que par l'odorat. En particulier, il ne produira pas de sensation sur l'organe du goût et n'aura donc pas de saveur. Aussi, la qualité d'un arôme en tant que matière susceptible d'augmenter l'appétissance est limitée.

La Demande de Brevet EP 0 320 320 décrit un comprimé pour animal domestique caractérisé en ce qu'il est constitué par au moins un noyau contenant un ou plusieurs principes actifs totalement englobés dans une matrice appétante pour l'animal. L'appétissance du comprimé est conférée par des agents tels que la poudre de foie ou la levure de bière qui sont des constituants de la matrice qui contient, par ailleurs, des excipients de compression et/ou des agents lubrifiants dont le rôle est de faciliter la fabrication du comprimé.

Cependant, la préparation de ces comprimés demande une étape de compression pour englober le noyau comprenant les principes actifs, ce qui constitue un inconvénient en ce qu'elle est susceptible d'endommager le ou les principes actifs. Par ailleurs, le ou les principes actifs se retrouvent être en contact intime avec les constituants appétants de la matrice qui ne sont pas nécessairement compatibles pour assurer une bonne stabilité aussi bien aux principes actifs qu'aux autres constituants de la matrice, notamment les constituants appétants de la matrice. De plus cette technologie de double compression (noyau d'une part et matrice appétante d'autre part) est délicate à mettre en oeuvre, en effet il est difficile de bien centrer le noyau dans la matrice appétante. Cette technologie est inapplicable aux comprimés de petite taille, par ailleurs adaptés pour les petites races de chiens ou les chats.

La Demande de Brevet EP 0 725 570 de la demanderesse décrit une composition attractive pour l'animal sous forme solide de volume suffisamment important pour ne pas être avalée mais mâchée par l'espèce cible, comprenant de 3 à 20% d'un polymère insoluble dans l'eau, de 5 à 45% en poids d'une substance appétissante et de 35 à 60% en poids de substance lipidique obtenue par fusion des substances lipidiques sous forme solide à une température inférieure à celle du point de fusion du/des polymères et mélange du/des polymères aux autres composants à la même température. La composition peut comprendre jusqu'à 50% en poids de substance bioactive et être mise sous forme de blocs. Il s'agit d'une formulation présentant une grande résistance mécanique pour des médicaments susceptibles d'être disséminés dans la nature pour traiter, par exemple, des animaux sauvages. La substance appétissante est répartie uniformément dans la forme galénique résultante.

La préparation de telles compositions nécessite un chauffage compris entre 40 et 80°C pour obtenir la fusion de certains constituants. Ce chauffage peut cependant constituer un inconvénient majeur pour la stabilité de certains principes actifs.

Les limites des formulations dans lesquelles les arômes et/ou les matières appétissantes et les principes actifs sont mélangées et donc en contact sont :
- qu'elles peuvent conduire à des incompatibilités entraînant la transformation ou dégradation du ou des principes actifs ou des arômes et des matières appétissantes. De plus, même présentes en quantité importante, les matières appétissantes ne masquent pas toujours l'odeur ou le goût désagréable à l'animal qui a un odorat et un goût très développés ; cela est d'autant plus vrai si la formulation contient des lipides, liquides ou solides, qui sont connus pour exalter les saveurs ;
- que la répartition homogène de la matière appétissante à l'intérieur des compositions rend la majorité de la matière appétissante indisponible pour exercer l'appétissance maximale avant que l'animal n'ait la formulation en bouche et ne commence à la mastiquer pour en ressentir le goût, le cas échéant ; et
- que ces formulations ne permettent pas toujours de protéger le principe actif des conditions environnementales telles que l'humidité et la température et de maintenir une parfaite stabilité dans le temps.

### 3. Encore une autre option pour faciliter l'administration orale de principes actifs est d'enrober le médicament dans des matières appétissantes.

Les Demandes de Brevet suivantes : FR 2 715 803, US 5,853,757, US 6,143,316, US 5,792,470, US 5,674,515, EP 0 574 301, US 4,857,333, DE 198 53 729, WO 03/030863, WO 2004/043427, WO 2007/090987, proposent des leurres réalisés avec des matières appétissantes dans lesquels on peut introduire extemporanément un médicament sous forme solide afin de l'administrer oralement à l'animal. Ces systèmes présentent l'avantage de s'adapter à de nombreuses formes solides de médicaments vétérinaires pour la voie orale.

Selon un principe similaire, la Demande WO 89/12442 décrit un support de composition vétérinaire pour poisson comprenant une couche externe imperméable à l'eau et aux principes actifs ; cette couche externe enveloppe au moins une chambre contenant au moins un principe actif et masque le goût dudit principe actif. La couche externe est constituée d'un matériau végétal ou animal, de préférence, de la farine de poisson, et/ou d'un extrait aqueux de matériau marin, et éventuellement d'un exhausteur de goût et/ou d'un liant. Une caractéristique importante de ce support de composition vétérinaire pour poisson est son caractère imperméable, notamment à l'eau, qui est obtenu par l'utilisation d'un liant dérivé d'amidon et par la présence de l'extrait aqueux de matériau marin qui agit comme une colle naturelle.

L'inconvénient commun de ces leurres est que leur utilisation nécessite l'opération préalable d'introduction du médicament dans le leurre, ce qui peut rebuter certains utilisateurs et aussi devenir gênant lorsqu'un grand nombre d'animaux doit être traité. De plus, leur volume important (nécessairement plus gros que le médicament) demande une quantité de matière importante et leur fabrication doit être adaptée à leur forme complexe ; ainsi ces leurres s'avèrent souvent coûteux.

La Demande de Brevet EP 0 725 627 de la demanderesse décrit une forme galénique pour l'administration orale de substances bioactives (biologiques, chimiques ou médicamenteuses) du type appât, attractive pour l'animal sous forme solide de volume suffisamment important pour ne pas être avalé mais mâché par l'espèce cible. Il s'agit d'une forme galénique à double compartiment comprenant :
(i) à titre de premier compartiment, un noyau central solide poreux contenant une ou plusieurs substances bioactives ; ce noyau a une composition particulière adaptée à une solidification par lyophilisation et il présente la caractéristique de se dissoudre ou de se désintégrer rapidement dans la salive ; ainsi, lorsque l'animal mâche ce médicament, les fragments générés par le noyau vont adhérer aux tissus de la cavité buccale de l'animal ; et
(ii) un second compartiment qui est une couche externe hydrophobe contenant de 3 à 30% d'une substance appétissante naturelle ou synthétique du type farine de viande ou de poisson, de 40 à 93% d'une substance lipidique et de 4 à 30% de polymère. La composition du second compartiment a comme spécificité de comporter des lipides afin de la rendre hydrophobe et de protéger le noyau contre l'eau, elle comporte également des polymères qui sont une charge structurelle ayant pour rôle de consolider la couche lipidique, de faciliter la manipulation de l'appât et de modifier le point de fusion de la composition ; cette couche est ainsi hydrophobe, appétissante et d'épaisseur contrôlée. Cependant, la préparation d'une telle formulation impose des contraintes mécaniques et thermiques défavorables à la stabilité de certains principes actifs.

Dans les présentations "gélules dures" ou "capsules molles", formes usuellement rejetées par l'animal, il est possible d'ajouter des arômes dans la tunique (paroi des gélules ou des capsules). La teneur en arôme est cependant nécessairement faible en raison des constituants de la tunique et de son poids faible par rapport au poids total des gélules ou capsules pleines. Ainsi, la présence d'arôme n'est souvent pas suffisante pour exercer une appétissance souhaitée et favoriser la prise, d'autant plus que l'arôme n'est perçu que par l'odorat, il ne produit pas de sensation sur l'organe du goût et n'a donc pas de saveur. Par ailleurs, la quantité d'arôme, sous forme liquide, introduite ne peut qu'être faible (généralement inférieure à 2 pour cent), et doit être compatible avec la nature du composant de la tunique qui est dans la majorité des cas la gélatine.

La Demande de Brevet EP 0 025 226 décrit des compositions d'enrobage de diverses formes solides alimentaire ou pharmaceutique à usage humain ; lesdites compositions sont constituées de saccharose, d'au moins un autre sucre, tel que le lactose, d'eau et peuvent en outre comprendre des colorants, agents de saveur, parfums et adjuvants. Ces compositions facilitent la mise en oeuvre d'un procédé d'enrobage par dragéification grâce à une composition en sucres qui les rend moins susceptibles de cristalliser en cours de procédé. L'enrobage en sucre, comme dans toutes les dragées, confère un goût agréable pour l'homme. Ce type de formulation n'est cependant pas adapté aux animaux ; les sucres purifiés n'étant pas une matière appétissant pour les animaux ; en outre, ils ne dégageant pas une odeur nécessaire à provoquer l'appétissance des animaux. Enfin, la forme "dragée" est mal acceptée par les animaux. Cette forme extrêmement dure et de surface très lisse est difficilement préhensible par les animaux.

En outre, une telle composition d'enrobage n'est pas compatible avec un éventuel ajout de matière appétissante pour animaux sous forme pulvérulente car :
- le sirop de sucre fortement chargé en matière appétissante est susceptible de provoquer un mauvais écoulement dans les buses de dragéification au cours du procédé de préparation des dragées ;
- la cristallisation du sucre lors de l'évaporation du solvant lors de la préparation des dragées aura pour effet de piéger la matière appétissante, diminuant ainsi considérablement l'attrait de la matière appétissante pour l'animal.

FR 2350105 décrit des préparations vétérinaires dont l'ingestion par des animaux est facilitée par l'utilisation d'une matière odorante et/ou gustative spécifique de l'animal auquel est destinée la préparation.

Il existe donc un besoin d'améliorer l'appétissance de médicaments solides administrés par voie orale aux animaux.

Ce besoin est particulièrement important pour les médicaments qui nécessitent une administration fréquente et répétée. C'est le cas en particulier pour les médicaments destinés à prévenir ou à traiter des pathologies chroniques. Ils peuvent devoir être administrés par exemple quotidiennement, une ou plusieurs fois par jour. Si le protocole de traitement prévoit par ailleurs que l'administration du médicament oral doit être décalée par rapport à la prise alimentaire, alors il est encore plus important que le médicament soit appétissant car il sera impossible de dissimuler la composition dans l'aliment pour faciliter sa prise.

Une catégorie importante de médicaments destinés à prévenir ou à traiter des maladies chroniques est celle des médicaments utilisés en cas d'insuffisance cardiaque. Ces pathologies ont une importance considérable en médecine vétérinaire et il existe un besoin constant d'améliorer les traitements.

La demanderesse s'est donné pour but de pallier les inconvénients de l'Art Antérieur et de mettre au point une composition et un procédé permettant de conférer aux médicaments à administration orale sous forme solide pour la prévention et/ou le traitement de l'insuffisance cardiaque chez les animaux une meilleure appétissance que les compositions et techniques connues. Elle s'est en particulier employée à mettre au point une composition vétérinaire à administration orale simple, économique, utilisable quelle que soit la forme galénique solide mise en jeu et dont la fabrication soit facilement industrialisable. En particulier, le traitement pour améliorer l'appétissance du médicament doit pouvoir être appliqué en fin de chaîne de fabrication dudit médicament, tout en assurant la stabilité chimique et physique de celui-ci.

Dans les travaux qui ont conduit à la mise au point de la composition d'enrobage selon l'invention, la demanderesse a constaté que l'adjonction d'une substance appétissante pour l'animal cible, se présentant sous forme pulvérulente à l'état de matière première, en quantité concentrée à la surface d'une composition solide administrée par voie orale, favorisait d'une part l'appétence de l'animal, et d'autre part incitait l'animal à consommer ladite composition solide. Les substances qui confèrent l'appétissance à une composition solide administrée par voie orale dépendent des goûts et des odeurs perçues et appréciées par l'animal cible.

L'invention se fonde sur cette constatation pour proposer un médicament appétissant à administration orale pour la prévention et/ou le traitement de l'insuffisance cardiaque chez les animaux très bien accepté par les animaux, absorbé rapidement, qu'il soit donné de façon occasionnelle ou répétée.

Les composés des classes thérapeutiques suivantes sont classiquement utilisés, dans le traitement de l'insuffisance cardiaque : les inhibiteurs de l'enzyme de conversion de l'angiotensine, les inhibiteurs des phosphodiestérases, les antagonistes de l'aldostérone et enfin les diurétiques. D'autres classes thérapeutiques importantes sont aussi utilisées dans le traitement de l'insuffisance cardiaque mais n'ont pas nécessairement d'applications homologuées par l'autorité administrative pour un usage en médecine vétérinaire. On peut citer à titre d'exemple, les glycosides cardiotoniques, les dérivés nitrés, les sympathicomimétiques.

Concernant plus précisément les Inhibiteurs de l'Enzyme de Conversion de l'Angiotensine (IECA), on trouve des spécialités à base des agents thérapeutiques suivants : Bénazépril ; Enalapril ; Imidapril ; Ramipril. Les IECA sont indiqués dans le cas d'une insuffisance cardiaque symptomatique débutante (grade II selon la classification NYHA), modérée (grade III) ou sévère (grade IV), le plus fréquemment suite à une insuffisance valvulaire (mitrale ou tricuspide) ou une cardiomyopathie dilatée. Ils inhibent l'enzyme de conversion responsable de la transformation de l'angiotensine I en angiotensine II qui est la seule forme active. L'enzyme de conversion intervient également dans la dégradation de la bradykinine. Du fait des effets vasoconstricteurs de l'angiotensine II, les IECA induisent un effet vasodilatateur artériel et veineux. Les IECA inhibent également la rétention d'eau et la vasoconstriction induites par la vasopressine ainsi que la rétention d'eau et de sodium et l'élimination de potassium contrôlées par l'aldostérone, deux hormones stimulées par l'angiotensine II. Ces effets hypotenseurs se font aux dépens d'une activation de la rénine et d'une accumulation plasmatique de l'angiotensine I. Les IECA peuvent être associés aux autres traitements conventionnels de l'insuffisance cardiaque. Ainsi, lorsque différents traitements nécessitant une prise orale solide sont associés, il est important que l'animal accepte facilement la prise de multiples formes orales.

Concernant les Inhibiteurs de phosphodiestérases, on trouve des spécialités à base de pimobendan. Le pimobendan est indiqué dans le traitement de l'insuffisance cardiaque suite à une cardiomyopathie ou à une insuffisance valvulaire (mitrale et/ou tricuspide). Les inhibiteurs des phosphodiestérases de type III exercent un effet inotrope positif et possèdent des propriétés vasodilatatrices exercées au niveau artériel et veineux conduisant ainsi à une réduction de la précharge et de la postcharge. L'inhibition de la dégradation de l'AMP cyclique et l'augmentation de la sensibilité calcique des fibres cardiaques contribuent à ces effets. Certaines de ces molécules ont une très grande affinité pour le système cardio-vasculaire, du fait de leur sélectivité pour un iso-enzyme particulier de la phosphodiestérase. Les effets inotropes positifs du pimobendan sont observés pendant 8 à 12 heures après l'administration orale. Après administration orale, la biodisponibilité du pimobendan est de 60 à 63%. Cette biodisponibilité est considérablement réduite lorsque le principe actif est administré pendant ou peu de temps après le repas. Ainsi est-il recommandé de ne pas nourrir le chien dans l'heure qui suit l'administration de la spécialité. Le pimobendan peut être utilisé chez le chien simultanément à d'autres traitements conventionnels de l'insuffisance cardiaque. Ainsi, lorsque différents traitements nécessitant une prise orale solide sont associés, il est important que l'animal accepte facilement la prise de multiples formes orales.

Concernant les antagonistes de l'aldostérone, on trouve des spécialités à base de spironolactone qui sont à utiliser en association avec une thérapie standard (incluant éventuellement un traitement diurétique), pour le traitement de l'insuffisance cardiaque congestive due à une régurgitation valvulaire, chez les chiens. La spironolactone et ses métabolites actifs sont des antagonistes spécifiques de l'aldostérone, qui se fixent de manière compétitive aux récepteurs minéralocorticoïdes situés dans les reins, le coeur et les vaisseaux sanguins. Dans les reins, la spironolactone inhibe la rétention de sodium induite par l'aldostérone, entraînant ainsi une augmentation de l'excrétion du sodium et par conséquent de l'eau et de la rétention du potassium. Ces effets rénaux conduisent à une diminution du volume extracellulaire et par conséquent à une diminution de la précharge cardiaque et de la pression de l'atrium gauche. II en résulte une amélioration de la fonction cardiaque. Dans le système cardiovasculaire, la spironolactone prévient les effets néfastes de l'aldostérone. Bien que son mécanisme d'action précis ne soit pas clairement défini, l'aldostérone favorise la fibrose myocardique, le remodelage myocardique et vasculaire et un dysfonctionnement endothélial. Des modèles expérimentaux sur des chiens ont montré qu'une thérapie à long terme avec un antagoniste de l'aldostérone prévient le dysfonctionnement progressif du ventricule gauche et atténue le remodelage du ventricule gauche chez les chiens présentant une insuffisance cardiaque chronique. Une étude clinique a démontré une réduction de la détérioration de l'état de l'animal atteint d'une maladie cardiovasculaire chez des chiens traités avec de la spironolactone en plus d'une thérapie standard (IECA) par rapport aux chiens traités uniquement avec une thérapie standard. Utilisé en en association avec des IECA, la spironolactone peut neutraliser l'effet d'échappement de l'aldostérone. La prise simultanée d'aliments augmente considérablement la biodisponibilité de la spironolactone. Après une administration orale de 2 à 4 mg/kg, l'absorption augmente linéairement avec la quantité administrée. Après de multiples administrations orales de 2 mg de spironolactone par kg pendant 10 jours consécutifs, aucune accumulation n'est observée. La distribution se fait principalement vers le tractus gastro-intestinal, les reins, le foie et les glandes surrénales. La spironolactone est rapidement et complètement métabolisée par le foie en 7α-thiométhylespironolactone et en canrénone, les principaux métabolites actifs chez le chien. Les Cmax sont atteintes après respectivement 2 et 4 heures pour les métabolites primaires, la 7α-thiométhyle-spironolactone et la canrénone. Un état d'équilibre est atteint au bout de 2 jours. La spironolactone est principalement excrétée sous forme de métabolites. Chez le chien, 70% de la dose administrée est retrouvée dans les fèces et 20 % dans l'urine. Il apparaît donc à nouveau que lorsque différents traitements nécessitant une prise orale solide sont associés, il est important que l'animal accepte facilement la prise de multiples formes orales.

La présente invention a en premier lieu pour objet l'utilisation d'une composition d'enrobage contenant une quantité adéquate d'une matière appétissante apte à être appliquée par un procédé de pelliculage (consistant à appliquer une fine couche de la composition d'enrobage en surface) pour l'enrobage par pelliculage d'une composition vétérinaire solide à administration orale indiquée en cas d'insuffisance cardiaque, c'est-à-dire recommandée et prescrite par le vétérinaire pour la et/ ou le traitement de l'insuffisance cardiaque chez les animaux, en particulier chez les carnivores domestiques et encore plus particulièrement chez le chien ou le chat.

La composition pharmaceutique vétérinaire solide à administration orale selon l'invention est un médicament pour usage vétérinaire destiné au traitement d'un état pathologique lié à une insuffisance cardiaque d'un animal, en particulier chez un chien ou un chat.

Ainsi, la présente invention se rapporte à l'utilisation d'une composition d'enrobage apte à être appliquée par un procédé de pelliculage sur une composition pharmaceutique vétérinaire solide à administration orale comprenant au moins un composé utilisé dans le traitement de l'insuffisance cardiaque, ladite composition pharmaceutique étant indiquée pour la prévention et/ou le traitement de l'insuffisance cardiaque chez les animaux, ladite composition d'enrobage étant caractérisée en ce :
(i) que la composition d'enrobage comprend :
   - une matière appétissante pour l'animal cible, se présentant sous forme pulvérulente, choisie parmi les substances d'origine animale ou végétale, directement mises en poudre après traitements tels que le séchage ou la déshydratation, le broyage, le calibrage mais aussi après transformation avec ajout d'autres composants pour favoriser la conservation par exemple. Parmi les substances de choix qui ont une forte appétissance pour les espèces cibles, en particulier les carnivores domestiques tels que les chiens et les chats, on trouve de manière non limitative la levure de bière, les farines de viandes, les farines de poissons, les poudres de fromages ou dérivés du lait, la poudre de foie et leur mélange ;
   - au moins un liant choisi parmi les polymères d'alcool polyvinylique, de polyvinylpyrrolidone, les copolymères de vinylpyrrolidone et d'acétate de vinyle, l'acétophthalate de polyvinyle, les celluloses et leurs dérivés, l'acide alginique et ses sels, la zeïne, l'acide hyaluronique, les pectines, la gomme arabique, la gomme adragante, la gomme karaya, la gomme xanthane, les carraghénanes, les polymères pullulan ou agar, le chitosan ou ses dérivés, les carbomères, l'acide acrylique réticulé avec des polyalkényl éthers, les polycarbophiles, les copolymères de méthylvinyl et d'anhydride maléique, les poly-saccharides ou le mélange d'au moins deux de ces liants ; étant entendu que lorsque le liant comprend un ou plusieurs polysaccharides, le pourcentage en poids du ou desdits dit polysaccharides représente 50% ou moins, de préférence 25% ou moins, du poids total de liants ; et
   - un solvant choisi parmi l'eau, le méthanol, l'éthanol, l'isopropanol, le propylène glycol, la glycérine ou leur mélange ;
(ii) qu'elle contient entre 30% et 90% inclus en poids de matière appétissante par rapport au poids total du mélange constitué par le liant et la matière appétissante ; et
(iii) que ladite composition vétérinaire solide à administration orale est indiquée pour la prévention et/ou le traitement de l'insuffisance cardiaque chez les animaux.

La matière appétissante pour l'animal cible se présente avantageusement sous forme pulvérulente, c'est-à-dire qu'elle est constituée de particules de petite taille ; cette forme contribue significativement au maintien du caractère appétissant pour l'animal de la matière appétissante même après l'enrobage par pelliculage de ladite matière sur une composition pharmaceutique vétérinaire solide à administration orale. En effet, la forme pulvérulente présente, par rapport aux formes liquides notamment, l'avantage de favoriser la restitution de particules et donc de maximiser les surfaces d'échanges avec l'extérieur après application de la composition d'enrobage sur la composition pharmaceutique solide vétérinaire à enrober.

L'attractivité pour l'animal de la composition pharmaceutique solide vétérinaire enrobée dépendra plus particulièrement de la charge importante de la composition d'enrobage en matière appétissante, de sa localisation en surface de la composition pharmaceutique solide vétérinaire et d'une surface d'échange la plus importante possible grâce à la présence de particules de petite taille. Le diamètre moyen des particules constituant la matière appétissante pour l'animal cible, se présentant sous forme pulvérulente, sera avantageusement inférieur à 500 µm, préférentiellement inférieur à 400 µm et encore plus préférentiellement compris entre 50 et 100 µm.

On pourra utiliser comme matière appétissante toute matière alimentaire qui provoque un attrait pour un animal cible ; ledit attrait pouvant être évalué par un test d'appétence.

Le solvant est utilisé pour mettre en solution ou en suspension le liant et la matière appétissante pulvérulente ; sa teneur est choisie par l'homme du métier en fonction de la nature dudit liant et de ladite matière appétissante pulvérulente. Le rapport poids de solvant sur poids total du mélange constitué par le liant et la matière appétissante est préférentiellement au moins supérieur à 2 et inférieure à 6 et encore plus préférentiellement compris entre 2 et 4.

Le choix du liant utilisé dans la composition d'enrobage doit rendre disponible la matière appétissante afin qu'elle exerce au mieux sa fonction sur l'animal. Il faut donc veiller à limiter le phénomène d'encapsulation de la matière appétissante sous forme pulvérulente dans des cristaux que pourraient former le liant lors du séchage en surface de la composition d'enrobage après son application sur la composition pharmaceutique solide vétérinaire.

Par celluloses et leurs dérivés, on entend notamment la cellulose microcristalline, les éthers ou esters des alkyl cellulose comme la méthyl cellulose, l'éthylcellulose, l'hydroxypropylcellulose, l'hydroxypropylméthylcellulose, l'acétophthalate de cellulose, l'acétate de cellulose.

Les poly-saccharides peuvent être la dextrine.

Lorsque des poly-saccharides sont utilisés en tant que liants, ils le sont en mélange avec au moins un liant d'une autre famille chimique (c'est-à-dire qui n'est ni un mono-, un di- ou un polysaccharide) et le pourcentage en poids des poly-saccharides n'excède pas 50%, préférentiellement 25%, du poids total des liants.

Les polyols peuvent être choisis parmi le sorbitol, le xylitol, l'isomalt, le maltitol, le mannitol, le lactitol ou leur mélange.

De préférence, la matière appétissante est de la poudre de foie, de la levure de bière ou un mélange de poudre de foie et de levure de bière.

De préférence, le liant utilisé est choisi parmi les celluloses ou encore leurs mélanges comprenant de préférence l'hydroxypropylméthylcellulose et la cellulose microcristalline.

De préférence, la composition d'enrobage contient entre 40% et 90% inclus en poids de matière appétissante par rapport au poids total du mélange constitué par le liant et la matière appétissante, plus préférentiellement elle contient entre 50% et 90%, encore plus préférentiellement entre 60% et 90% inclus en poids de matière appétissante par rapport au poids total du mélange constitué par le liant et la matière appétissante. De manière encore plus préférentielle, la composition d'enrobage contient entre 60% et 80% inclus en poids de matière appétissante par rapport au poids total du mélange constitué par le liant et la matière appétissante.

Ainsi composée de ces trois ingrédients : matière appétissante, liant et solvant, la composition d'enrobage peut être utilisée à l'exclusion de tout autre ingrédient.

Selon une autre variante de l'invention, la composition d'enrobage comprend en outre un ou des additifs acceptables choisis, à titre d'exemple et sans aucun caractère limitatif, parmi les plastifiants comme l'acide stéarique et ses dérivés, l'acide citrique et ses dérivés, l'acide lactique et ses dérivés, le propylène glycols, la glycérine, les phthalates et leurs dérivés, les adipates et leurs dérivés, les sébaçates et leurs dérivés, les polyéthylène glycols et leurs dérivés,; des stabilisants comme les antioxydants ou des conservateurs tels que l'acide ascorbique et ses dérivés ou sels, le butylhydroxyanisol, le butylhydroxytoluène, l'acide gallique et ses dérivés, le métabisulfite de sodium ou potassium, le bisulfite de sodium, les vitamines et leurs dérivés, l'EDTA et ses sels, les parabens ; des charges tels que le talc, la silice, les silicates, la cellulose microcristalline, le mica, les carbonates, les silicones ; des surfactants ; des colorants tels que les oxydes de fer, les colorants solubles absorbés sur laques d'alumine, l'oxyde de titane ; ou encore des porogènes.

L'invention se rapporte également à un procédé d'enrobage par pelliculage qui permet de fixer en surface d'une composition pharmaceutique vétérinaire solide à administration orale pour la prévention et/ou le traitement de l'insuffisance cardiaque chez les animaux une matière appétissante, préférentiellement une matière appétissante pulvérulente. Le pelliculage de la composition pharmaceutique vétérinaire solide à administration orale pour la prévention et/ou le traitement de l'insuffisance cardiaque chez les animaux est obtenu selon les techniques et les matériels classiques connus de l'homme de métier.

Plus particulièrement, la présente invention se rapporte à un procédé de préparation d'une composition pharmaceutique vétérinaire solide à administration orale pour la prévention et/ou le traitement de l'insuffisance cardiaque chez les animaux enrobée d'une matière appétissante (ci-après désignée composition appétissante à administration orale pour la prévention et/ou le traitement de l'insuffisance cardiaque chez les animaux), caractérisé en ce qu'il comprend les étapes suivantes :
(1) application sur une composition pharmaceutique vétérinaire solide à administration orale d'une couche d'une composition d'enrobage comprenant au moins une matière appétissante pour l'animal cible sous forme pulvérulente, un liant et un solvant ;
(2) séchage de ladite composition enrobée obtenue à l'étape (1) ;
(3) optionnellement, répétition des étapes (1) et (2) ; et
(4) obtention d'une composition appétissante à administration orale pour la prévention et/ou le traitement de l'insuffisance cardiaque chez les animaux dans laquelle ladite matière appétissante représente au moins 5% et de préférence au moins 15% en poids de ladite composition appétissante à administration orale pour la prévention et/ou le traitement de l'insuffisance cardiaque chez les animaux.

Selon un mode de réalisation particulier, le procédé selon l'invention est caractérisé en ce qu'il comprend les étapes suivantes :
(A) introduction d'une composition pharmaceutique vétérinaire solide à administration orale pour la prévention et/ou le traitement de l'insuffisance cardiaque chez les animaux à enrober dans une turbine à dragéifier ;
(B) pulvérisation à l'aide d'une buse d'une composition d'enrobage comprenant au moins une matière appétissante pour l'animal cible sous forme pulvérulente, un liant et un solvant, dans ladite turbine en rotation en une quantité telle que ladite matière appétissante représente au moins 5% et de préférence au moins 15% en poids de la composition appétissante pharmaceutique vétérinaire solide à administration orale pour la prévention et/ou le traitement de l'insuffisance cardiaque chez les animaux.

Les conditions de température pour la mise en oeuvre du procédé sont propres à l'équipement et aux ingrédients utilisés. De façon avantageuse, la mise en oeuvre du procédé selon l'invention est telle que la température de la composition pharmaceutique vétérinaire solide à administration orale ne dépasse pas 42°C, de préférence 40°C et encore préférentiellement 38°C.

L'enrobage nécessite l'utilisation d'une composition d'enrobage comme définie ci-avant, qui est une solution ou une suspension aqueuse ou organique d'au moins un liant choisi à titre d'exemple et sans caractère limitatif parmi, les polymères d'alcool polyvinylique, de polyvinylpyrrolidone, les copolymères de vinylpyrrolidone et d'acétate de vinyle, l'acétophthalate de polyvinyle, les celluloses et leurs dérivés tels que les éthers ou esters des alkyl cellulose comme la méthylcellulose, l'éthylcellulose, l'hydroxypropylcellulose l'hydroxypropylméthylcellulose, l'acétophthalate de cellulose, l'acétate de cellulose, l'acide alginique et ses sels, la zeïne, l'acide hyaluronique, les pectines, la gomme arabique, la gomme adragante, la gomme karaya, la gomme xanthane, les carraghénanes, les polymères pullulan ou agar, le chitosan ou ses dérivés, les carbomères, l'acide acrylique réticulé avec des polyalkenyl éthers, les polycarbophiles, les copolymères de méthylvinyl et d'anhydride maléique, les poly-saccharides choisis parmi les dextrines; ou le mélange d'au moins deux de ces liants dans un solvant et contenant la matière appétissante.

Lorsque des poly-saccharides sont utilisés en tant que liants, ils le sont en mélange avec au moins un liant d'une autre famille chimique (c'est-à-dire qui n'est ni un mono-, un di- ou un polysaccharide) et le pourcentage en poids des mono-saccharides, di-saccharides et poly-saccharides n'excède pas 50%, préférentiellement 25%, du poids total des liants..

L'enrobage s'obtient par application de la solution ou suspension et évaporation du solvant. Cette application peut être répétée plusieurs fois de manière à obtenir le taux de matière appétissante voulu.

La composition d'enrobage selon l'invention est soit aqueuse soit organique avec des solvants tels que le méthanol, l'éthanol, l'isopropanol, le propylène glycol, la glycérine ou encore une solution hydroalcoolique.

La présente invention porte particulièrement sur une composition appétissante pharmaceutique vétérinaire solide à administration orale pour la prévention et/ou le traitement de l'insuffisance cardiaque chez les animaux comprenant comme composé actif, le pimobendan qui présente un goût ou une odeur désagréable qui gêne ainsi la prise du médicament vétérinaire par l'animal cible. Ce composé actif peut présenter une instabilité, par exemple vis-à-vis de la matière appétissante, ce qui gênerait l'obtention d'un médicament appétissant ainsi que la prise du médicament vétérinaire par l'animal cible par l'impossibilité d'utiliser cette matière appétissante.

La composition pharmaceutique vétérinaire solide à administration orale est, par exemple et sans que cette liste ait un caractère limitatif, un comprimé, une gélule dure, une capsule molle, une pilule, une tablette, des granulés, un cachet, une gomme à mâcher, une pastille.

Selon une variante de l'objet de l'invention, la composition appétissante pharmaceutique vétérinaire solide à administration orale pour la prévention et/ou le traitement de l'insuffisance cardiaque chez les animaux comprenant est une gélule dure à administration orale qui comprend au moins un excipient présentant un goût ou une odeur désagréable qui gêne ainsi la prise de la composition vétérinaire par l'animal cible et/ou un excipient présentant une instabilité, par exemple vis-à-vis de la matière appétissante, qui gêne l'obtention d'un médicament appétissant ainsi que la prise de la composition vétérinaire par l'animal cible.

Par excipient, on entend tout ingrédient de ladite composition pharmaceutique vétérinaire solide à administration orale. Il s'agit, par exemple et sans caractère limitatif, des charges, des conservateurs, des stabilisants, des colorants, des porogènes, des agents de désagrégation, des liants, des acidifiants, des agents d'écoulement tels que décrits dans le brevet allemand DE 4001622 ou le PCT WO 2005/084647.

En outre, la composition pharmaceutique vétérinaire solide à administration orale est optionnellement sécable. En effet, on s'est rendu compte que le fait de réaliser sur l'une ou les deux faces principales de la composition appétissante pharmaceutique vétérinaire solide à administration orale, une ou plusieurs, notamment deux, entailles faisant office de barre de sécabilité n'enlève rien à la facilité de la prise de ladite composition appétissante pharmaceutique vétérinaire solide à administration orale ou des morceaux obtenus après rupture suivant la ou les barres de sécabilité.

Ainsi, une variante de mise en oeuvre du procédé selon l'invention, consiste à utiliser une composition pharmaceutique vétérinaire solide à administration orale qui a une forme sécable, ce qui lui permet, après l'étape d'enrobage par pelliculage de pouvoir être divisée selon les besoins de l'animal à soigner.

Selon un autre mode avantageux de mise en oeuvre du procédé selon l'invention, la composition pharmaceutique vétérinaire solide à administration orale pour la prévention et/ou le traitement de l'insuffisance cardiaque chez les animaux est séparée de la composition d'enrobage contenant la matière appétissante par un film qualifié de film primaire. Ce film primaire peut être obtenu par une encapsulation, un enrobage ou un pelliculage de ladite composition pharmaceutique vétérinaire solide à administration orale pour la prévention et/ou le traitement de l'insuffisance cardiaque chez les animaux par un film, principalement polymérique, préalablement à l'enrobage par la composition d'enrobage contenant la matière appétissante. Ce film primaire peut avoir pour fonction de produire, selon les constituants choisis, une barrière isolante et protéger ainsi la composition pharmaceutique vétérinaire solide à administration orale pour la prévention et/ou le traitement de l'insuffisance cardiaque chez les animaux, et, en particulier, le ou les principes actifs, de toutes les attaques physiques ou chimiques susceptibles d'être induites par la composition d'enrobage selon l'invention. Le film primaire peut aussi avoir pour fonction de faciliter l'adhésion de la composition d'enrobage amenant la matière appétissante à la surface de la composition pharmaceutique vétérinaire solide à administration orale, Enfin, le film primaire peut avoir pour fonction à la fois de fournir une barrière isolante à la surface de la composition pharmaceutique vétérinaire solide à administration orale et de faciliter l'adhésion de la composition d'enrobage amenant la matière appétissante à la surface de la même composition pharmaceutique vétérinaire solide à administration orale.

Ainsi, le procédé selon l'invention peut comprendre une étape additionnelle préalable à l'étape (1) et qui consiste à recouvrir, c'est-à-dire à pelliculer ou enrober ladite composition pharmaceutique vétérinaire solide à administration orale pour la prévention et/ou le traitement de l'insuffisance cardiaque chez les animaux d'un film primaire à l'aide d'une solution ou suspension comprenant au moins un liant et au moins un solvant et/ou un ou des additifs acceptables pour l'effet recherché ; le liant, le solvant et les additifs acceptables sont tels que définis précédemment.

Tout comme pour l'application de la composition d'enrobage selon l'invention en surface de la composition pharmaceutique vétérinaire solide à administration orale pour la prévention et/ou le traitement de l'insuffisance cardiaque chez les animaux, l'application préalable du film primaire en surface de la composition pharmaceutique vétérinaire solide à administration orale est réalisée par des techniques classiques d'enrobage ou de pelliculage de compositions pharmaceutiques solides.

La présente invention a également pour objet une composition pharmaceutique vétérinaire solide à administration orale appétissante pour la prévention et/ou le traitement de l'insuffisance cardiaque chez les animaux contenant en tant qu'ingrédient actif préféré du pimobendan, caractérisée en ce qu'elle est susceptible d'être obtenue par le procédé d'enrobage par pelliculage selon l'invention.

Cette composition pharmaceutique vétérinaire solide appétissante à administration orale pour la prévention et/ou le traitement de l'insuffisance cardiaque chez les animaux est caractérisée en ce qu'elle comprend :
une composition pharmaceutique vétérinaire solide à administration orale pour la prévention et/ou le traitement de l'insuffisance cardiaque chez les animaux contenant au moins un principe actif ; et
   - une composition d'enrobage positionnée autour de ladite composition pharmaceutique vétérinaire solide à administration orale pour la prévention et/ou le traitement de l'insuffisance cardiaque chez les animaux d'une matière appétissante et d'un liant ; ladite composition d' enrobage contenant entre 30% et 90% inclus en poids de matière appétissante par rapport au poids total de l'enrobage constitué par le liant et la matière appétissante ; ladite matière appétissante représentant au moins 5% et préférentiellement au moins 15% en poids de ladite composition pharmaceutique vétérinaire solide appétissante à administration orale pour la prévention et/ou le traitement de l'insuffisance cardiaque chez les animaux.

La matière appétissante et le liant sont tels que définis ci-dessus.

Le principe actif préféré de la composition pharmaceutique vétérinaire solide à administration orale pour la prévention et/ou le traitement de l'insuffisance cardiaque chez les animaux est le pimobendan.

La forme préférée de la composition pharmaceutique vétérinaire solide à administration orale pour la prévention et/ou le traitement de l'insuffisance cardiaque chez les animaux est une gélule dure.

L'avantage que présente cette forme particulière est qu'elle conduit à une composition pharmaceutique vétérinaire solide appétissante à administration orale pour la prévention et/ou le traitement de l'insuffisance cardiaque chez les animaux est que la matière appétissante est concentrée en surface de ladite gélule dure appétissante à administration orale. Ainsi en utilisant une quantité limitée et économique de matière appétissante, on obtient néanmoins une très bonne efficacité de prise du médicament appétissant comme cela est démontré dans les exemples qui suivent.

Cette composition pharmaceutique vétérinaire solide appétissante à administration orale pour la prévention et/ou le traitement de l'insuffisance cardiaque chez les animaux trouve un intérêt particulier pour l'administration du pimobendan mal acceptés par les animaux. Elle peut cependant être utilisée pour favoriser l'administration de tout autre actif d'intérêt vétérinaire pour la prévention et/ou le traitement de l'insuffisance cardiaque chez les animaux dans la mesure où elle facilite la prise de la composition vétérinaire et elle favorise la conservation de l'actif.

D'une manière générale, la composition pharmaceutique vétérinaire solide appétissante à administration orale pour la prévention et/ou le traitement de l'insuffisance cardiaque chez les animaux contient entre 0,01 et 50% inclus en poids par rapport au poids total de la composition pharmaceutique vétérinaire solide appétissante à administration orale pour la prévention et/ou le traitement de l'insuffisance cardiaque chez les animaux de pimobendan.

D'une manière préférée, la teneur en principe actif ne dépasse pas 35% en poids du poids de la composition pharmaceutique vétérinaire solide appétissante à administration orale pour la prévention et/ou le traitement de l'insuffisance cardiaque chez les animaux.

Le pimobendan peut être simplement répartis au sein de la composition pharmaceutique vétérinaire solide à administration orale

Le pimobendan peut être lui-même encapsulé ou enrobé par les techniques connues de l'homme du métier afin d'améliorer sa stabilité ou d'augmenter le masquage de son odeur et de son goût à la perception olfactive ou gustative de l'animal.

L'invention concerne aussi l'utilisation de pimobendan pour la fabrication d'une composition appétissante pharmaceutique vétérinaire solide à administration orale pour la prévention et/ou le traitement de l'insuffisance cardiaque chez les animaux selon l'invention.

D'une manière préférée, la composition pharmaceutique vétérinaire solide appétissante à administration orale pour la prévention et/ou le traitement de l'insuffisance cardiaque chez les animaux est une gélule dure.

La composition appétissante pharmaceutique vétérinaire solide à administration orale est préférentiellement destinée à un animal carnivore domestique tel qu'un chien ou un chat.

Les exemples suivants, non limitatifs, illustrent l'invention.

### EXEMPLE 1 - Préparation de gélules dures enrobées selon l'invention

Pour encapsuler 765 g de gélules dure de 130 mg contenant un mélange de pimobendan, acide citrique anhydre, silice colloïdale anhydre, cellulose microcristalline, povidone et stéarate de magnésium, on prépare la dispersion de matière appétissante à pulvériser suivante :
- eau 452 g
- hydroxypropylmethylcellulose / cellulose microcristalline / macrogol stéarate 45 g
- Poudre de foie de volaille 115 g

Les gélules dures sont introduites dans la turbine à dragéifier où elles sont dépoussiérées et mises en température.

La suspension est au préalable filtrée sur un tamis de 500 µm.

L'encapsulation est effectuée pendant environ 2h15 avec un débit croissant de 3,5 à 4,6 g/min à l'aide d'une buse de 1,8 mm.

La pulvérisation de la suspension de matière appétissante est arrêtée quand la quantité requise de matière appétissante est atteinte.

La température de l'air entrant est maintenue à environ 55°C, en vérifiant que la température des gélules dures ne dépasse pas 38 - 40°C.

En fin d'opération d'enrobage la température des gélules dures enrobées est ramenée à 25°C.

La quantité de matière appétissante, la poudre de foie de volaille, déposée sur la gélule dure est calculée comme suit :
- La masse de 50 gélules dures non enrobées est de 6,50 g soit pour une gélule dure, 130 mg, et
- La masse de 50 gélules dures enrobées est de 8,38 g soit pour une gélule dure enrobée, 167,6 mg,

Ainsi le poids de l'enrobage est de 37,6 mg constitué de 10,53 mg (28% m/m du poids de l'enrobage) de liants et 27,07 mg (72% m/m du poids de l'enrobage) de matière appétissante soit 16,15% p/p de matière appétissante du poids total de gélule dure enrobée.

### EXEMPLE 2 - test monadique d'appétence et de stabilité avec les gélules dures enrobées de l'exemple 1

### a) Appétence

Un test d'appétence des gélules dures appétissantes à administration orale obtenues dans l'exemple 1 et du médicament non enrobé (gélules n°3 de 130 mg contenant du pimobendan) a été réalisé auprès de trente six chiens adultes, mâles et femelles, de races variées en test croisé.

Le premier jour, 18 chiens ont testé les gélules dures enrobées de l'exemple 1 et 18 chiens ont testé les gélules non enrobées. Les chiens de moins de 20 kg de poids corporel de chacun des deux groupes ont reçu 1 gélule dure enrobée ou 1 gélule dure non enrobée et les chiens de plus de 20 kg de chacun des deux groupes ont reçu 2 gélules dures enrobées ou 2 gélules dures non enrobées.

Le troisième jour, le premier groupe de 18 chiens a testé les gélules dures non enrobées tandis que le second groupe de 18 chiens a testé les gélules dures enrobées de l'exemple 1. Les chiens de moins de 20 kg de poids corporel de chacun des deux groupes ont reçu 1 gélule dure enrobée ou 1 gélule dure non enrobée et les chiens de plus de 20 kg de chacun des deux groupes ont reçu 2 gélules dures enrobées ou 2 gélules dures non enrobées.

Il s'agit d'un test monadique réalisé sur deux journées de test et mené dans des box individuels pendant dix minutes par chiens. Ont été mesurés :
- la préhension
   - à la main,
   - au sol, ou
   - pas de prise
- la consommation
   - partielle,
   - totale, ou
   - pas de consommation.

Pour chacun de ces critères est précisé le nombre d'individus, sur l'ensemble du panel et par catégories de taille (petite/moyenne/grande).

Le calcul de l'acceptabilité est basé sur le pourcentage de chiens ayant consommé la totalité des gélules dures enrobées ou non.

### Préhension :

**Gélules enrobées selon l'invention (exemple 1)**

| | petits chiens | chiens moyens | | grands chiens |
|---|---|---|---|---|
| | | < 20 kg | > 20 kg | |
| à la main | 12 | 7 | 3 | 9 |
| au sol | 0 | 0 | 1* | 3* |
| pas de prise | 0 | 0 | 1** | 0 |

**Gélules non enrobées**

| | petits chiens | chiens moyens | | grands chiens |
|---|---|---|---|---|
| | | < 20 kg | > 20 kg | |
| à la main | 7 | 4 | 2 | 4 |
| au sol | 3 | 2 | 1* | 3* |
| pas de prise | 2 | 1 | 1/1** | 3/2** |

| | | | | |
|---|---|---|---|---|
| * 1 gélule est prise à la main et la seconde au sol ** 1 gélule est prise à la main ou au sol et la seconde n'est pas prise | | | | |

Consommation : prise en compte des seuls animaux ayant pris le produit.

**Gélules enrobées selon l'invention (exemple 1)**

| | petits chiens | chiens moyens | | grands chiens |
|---|---|---|---|---|
| | | < 20 kg | > 20 kg | |
| partielle | 0 | 0 | 1 | 0 |
| totale | 12 | 7 | 3 | 12 |
| pas de consommation | 0 | 0 | 0 | 0 |

**Gélules non enrobées**

| | petits chiens | chiens moyens | | grands chiens |
|---|---|---|---|---|
| | | < 20 kg | > 20 kg | |
| partielle | 1*** | 0 | 2*** | 2*** |
| totale | 2/3**** | 2/2**** | 1**** | 3/1**** |
| pas de consommation | 4 | 2 | 0 | 1 |

| | | | | |
|---|---|---|---|---|
| *** Il y a lieu de mentionner le comportement des animaux avec ce type de support : en général, les chiens ont pris le support en gueule. Compte tenu du pouvoir adhésif de la gélule à la cavité buccale en présence de la salive dû à sa composition en gélatine, les animaux cherchent à recracher la ou les gélules ce qui a entraîné leur percement, qui a eu pour effet de disperser une partie du contenu de cette dernière au sol quand ce n'est pas la gélule qui était rejetée directement d'autant plus que l'animal secouait la tête, gueule vers le bas. Dans ce cas de figure, il a été attribué une valeur « consommation partielle » à ces cas. **** Comme ci-dessus, les chiens ont rejeté la gélule dure ou une partie de son contenu. Dans ce cas de figure, lorsque certains animaux ont léché tout ou partie de la composition médicamenteuse au sol, il a été attribué une valeur « consommation totale » à ces cas. | | | | |

Pour les gélules dures enrobées selon l'invention, l'acceptabilité a été de 97,22% et la consommation totale de 94,44%, alors que l'acceptabilité des gélules dures non enrobées a été de 77,22 % et leur consommation totale de 38,88%.

La prise simultanée de plusieurs gélules dures enrobées selon l'invention est de 94,11% et leur consommation totale est de 88,23%, alors que la prise simultanée des gélules dures non enrobées a été de 58,82% et leur consommation totale maximale de 29,41% en faisant l'approximation consistant à considérer que les animaux qui ont léchés la composition médicamenteuse au sol ont consommés la totalité de la dose administrée.

### EXEMPLE 3 - Préparation de gélules dures enrobées préalablement pelliculées selon l'invention

### 1- pelliculage avec un film primaire isolant

On prépare la dispersion à pulvériser, pour former un film primaire isolant, suivante :
- eau 140,25 g
- hydroxypropylmethylcellulose / cellulose microcristalline / acide stéarique 19,125 g
   pour encapsuler 765 g de gélules dure de 130 mg (de composition : pimobendan / acide citrique anhydre / silice colloïdale anhydre / cellulose microcristalline / povidone / stéarate de magnésium).

Les gélules dures sont introduites dans la turbine à dragéifier où elles sont dépoussiérées et mis en température.

L'enrobage est effectué pendant environ 2h avec un débit croissant de 3,1 à 4,56 g/min à l'aide d'une buse de 1 mm.

La température de l'air entrant est maintenue à environ 50°C, en vérifiant que la température des gélules ne dépasse pas 40 - 42°C.

En fin d'opération de pelliculage, la température des gélules dures pelliculées avec le film primaire isolant est ramenée à 25°C.

### 2- enrobage avec la matière appétissante

On prépare la dispersion de matière appétissante à pulvériser suivante :
- eau 570,0 g
- hydroxypropylmethylcellulose / cellulose microcristalline / macrogol stéarate 56,75 g
- Poudre de foie de volaille 145,0 g
   pour enrober les gélules dures pelliculées obtenues à l'étape 1 ci-dessus.

La suspension est au préalable filtrée sur un tamis de 500 µm.

L'enrobage est effectué pendant environ 5h avec un débit croissant de 3,5 à 4,6 g/min à l'aide d'une buse de 1,8 mm.

La température de l'air entrant est maintenue à environ 50 - 55°C, en vérifiant que la température des comprimés ne dépasse pas 38 - 40°C.

La pulvérisation de la suspension de matière appétissante est arrêtée quand la quantité requise de matière appétissante est atteinte.

En fin d'opération d'enrobage, la température des gélules dures enrobées de matière appétissante est ramenée à 25°C.

La quantité de matière appétissante, la poudre de foie de volaille, déposée sur la gélule dure est calculée comme suit :
- la masse de 50 gélules dures est de 6,5 g soit pour une gélule dure, 130 mg,
- la masse de 50 gélules dures pelliculée est de 6,65 g soit pour une gélule dure, 133 mg, et
- la masse de 50 gélules dures enrobées est de 8,42 g soit pour une gélule dure enrobée : 168,4 mg.

Ainsi le poids de l'enrobage est de 35,4 mg constitué de 9,97 mg (28,18 % m/m du poids de l'enrobage) de liant et 25,44 mg (71,87% m/m du poids de l'enrobage) de matière appétissante soit 15,10% p/p de matière appétissante du poids total de la gélule dure enrobée.

## Revendications

1. Utilisation d'une composition d'enrobage apte à être appliquée par un procédé de pelliculage sur une composition pharmaceutique vétérinaire solide à administration orale comprenant au moins un composé utilisé dans le traitement de l'insuffisance cardiaque, ladite composition pharmaceutique étant indiquée pour la prévention et/ou le traitement de l'insuffisance cardiaque chez les animaux, ladite composition d'enrobage étant **caractérisée** en ce :
(i) qu'elle comprend :
- une matière appétissante pour l'animal cible, se présentant sous forme pulvérulente et consistant en une substance d'origine animale ou végétale directement mise en poudre ;
- au moins un liant choisi parmi les polymères d'alcool polyvinylique, de polyvinylpyrrolidone, les copolymères de vinylpyrrolidone et d'acétate de vinyle, l'acétophthalate de polyvinyle, les celluloses et leurs dérivés, l'acide alginique et ses sels, la zeïne, l'acide hyaluronique, les pectines, la gomme arabique, la gomme adragante, la gomme karaya, la gomme xanthane, les carraghénanes, les polymères pullulan ou agar, le chitosan ou ses dérivés, les carbomères, l'acide acrylique réticulé avec des polyalkényl éthers, les polycarbophiles, les copolymères de méthylvinyl et d'anhydride maléique, les polysaccharides ou le mélange d'au moins deux de ces liants ; étant entendu que lorsque le liant comprend un ou plusieurs polysaccharides, le pourcentage en poids du ou desdits dit polysaccharides représente 50% ou moins, de préférence 25% ou moins, du poids total de liants ; et
- un solvant choisi parmi l'eau, le méthanol, l'éthanol, l'isopropanol, le propylène glycol, la glycérine ou leur mélange ;
et (ii) qu'elle contient entre 30% et 90% inclus en poids de matière appétissante par rapport au poids total du mélange constitué par le liant et la matière appétissante pour l'enrobage par pelliculage de ladite composition pharmaceutique vétérinaire solide à administration orale.

2. Utilisation d'une composition d'enrobage selon la revendication 1, **caractérisée en ce que** ladite composition vétérinaire solide à administration orale indiquée pour la prévention et/ou le traitement de l'insuffisance cardiaque chez les animaux est à base de pimobendan.

3. Utilisation d'une composition d'enrobage selon la revendication 1 ou 2, **caractérisée en ce que** ladite matière appétissante pulvérulente est choisie parmi la levure de bière, les farines de viandes, les farines de poissons, les poudres de fromages ou dérivés du lait, la poudre de foie et leur mélange.

4. Utilisation d'une composition d'enrobage selon l'une quelconque des revendications précédentes, **caractérisée en ce que** ladite matière appétissante pulvérulente est constituée de particules ayant un diamètre moyen inférieur à 500 µm, préférentiellement inférieur à 400 µm et plus préférentiellement compris entre 50 et 100 µm.

5. Utilisation d'une composition d'enrobage selon l'une quelconque des revendications précédentes, **caractérisée en ce qu'**elle comprend en outre au moins un additif choisi parmi les plastifiants ; des stabilisants ; des conservateurs ; des charges ; des surfactants ; des colorants et des porogènes.

6. Utilisation selon l'une quelconque des revendications précédentes, **caractérisée en ce que** ladite composition pharmaceutique vétérinaire solide à administration orale est destinée à un animal carnivore domestique.

7. Procédé de préparation d'une composition pharmaceutique vétérinaire solide à administration orale pour la prévention et/ou le traitement de l'insuffisance cardiaque chez les animaux comprenant au moins un composé utilisé dans le traitement de l'insuffisance cardiaque et enrobée par pelliculage d'une matière appétissante, **caractérisé en ce qu'**il comprend les étapes suivantes :
(1) application sur une composition pharmaceutique vétérinaire solide à administration orale pour la prévention et/ou le traitement de l'insuffisance cardiaque chez les animaux comprenant au moins un composé utilisé dans le traitement de l'insuffisance cardiaque d'une couche d'une composition d'enrobage comprenant :
- au moins une matière appétissante pour l'animal cible sous forme pulvérulente et consistant en une substance d'origine animale ou végétale directement mise en poudre ;
- au moins un liant choisi parmi les polymères d'alcool polyvinylique, de polyvinylpyrrolidone, les copolymères de vinylpyrrolidone et d'acétate de vinyle, l'acétophthalate de polyvinyle, les celluloses et leurs dérivés, l'acide alginique et ses sels, la zeïne, l'acide hyaluronique, les pectines, la gomme arabique, la gomme adragante, la gomme karaya, la gomme xanthane, les carraghénanes, les polymères pullulan ou agar, le chitosan ou ses dérivés, les carbomères, l'acide acrylique réticulé avec des polyalkényl éthers, les polycarbophiles, les copolymères de méthylvinyl et d'anhydride maléique, les polysaccharides ou le mélange d'au moins deux de ces liants ; étant entendu que lorsque le liant comprend un ou plusieurs polysaccharides, le pourcentage en poids du ou desdits dit polysaccharides représente 50% ou moins, de préférence 25% ou moins, du poids total de liants ; et
- un solvant choisi parmi l'eau, le méthanol, l'éthanol, l'isopropanol, le propylène glycol, la glycérine ou leur mélange ;
ladite composition d'enrobage étant telle qu'elle contient entre 30% et 90% inclus en poids de matière appétissante par rapport au poids total du mélange constitué par le liant et la matière appétissante ;
(2) séchage de ladite composition enrobée obtenue à l'étape (1) ;
(3) optionnellement, répétition des étapes (1) et (2) ; et
(4) obtention d'une composition appétissante pharmaceutique vétérinaire solide à administration orale pour la prévention et/ou le traitement de l'insuffisance cardiaque chez les animaux dans laquelle ladite matière appétissante représente au moins 5% et de préférence au moins 15% en poids de ladite composition appétissante pharmaceutique vétérinaire solide à administration orale.

8. Procédé selon la revendication 7, **caractérisé en ce que** ladite composition vétérinaire solide à administration orale pour la prévention et/ou le traitement de l'insuffisance cardiaque chez les animaux comprend du pimobendan.

9. Procédé selon l'une quelconque des revendications 7 ou 8, **caractérisé en ce que** ladite composition pharmaceutique vétérinaire solide à administration orale est un comprimé, une gélule dure, une capsule molle, une pilule, une tablette, des granulés, un cachet, une gomme à mâcher, une pastille.

10. Procédé selon la revendication 9, **caractérisé en ce que** ladite composition pharmaceutique vétérinaire solide à administration orale pour la prévention et/ou le traitement de l'insuffisance cardiaque chez les animaux est une gélule dure.

11. Procédé selon l'une quelconque des revendications 7 à 9, **caractérisé en ce que** ladite composition pharmaceutique vétérinaire solide à administration orale pour la prévention et/ou le traitement de l'insuffisance cardiaque chez les animaux est sécable.

12. Procédé selon l'une quelconque des revendications 7 à 11, **caractérisé en ce qu'**il comprend une étape additionnelle préalable à l'étape (1) consistant à enrober ladite composition pharmaceutique vétérinaire solide à administration orale d'un film primaire à l'aide d'une solution ou suspension comprenant au moins un liant et au moins un solvant et/ou un ou des additifs acceptables.

13. Composition appétissante pharmaceutique vétérinaire solide à administration orale pour la prévention et/ou le traitement de l'insuffisance cardiaque chez les animaux, **caractérisée en ce qu'**elle comprend :
- une composition pharmaceutique vétérinaire solide à administration orale contenant au moins un principe actif consistant en un composé utilisé dans le traitement de l'insuffisance cardiaque ; et
- un enrobage appliqué par pelliculage positionné autour de ladite composition constitué d'une matière appétissante se présentant sous forme pulvérulente consistant en une substance d'origine animale ou végétale directement mise en poudre et d'au moins un liant choisi parmi les polymères d'alcool polyvinylique, de polyvinylpyrrolidone, les copolymères de vinylpyrrolidone et d'acétate de vinyle, l'acétophthalate de polyvinyle, les celluloses et leurs dérivés, l'acide alginique et ses sels, la zeïne, l'acide hyaluronique, les pectines, la gomme arabique, la gomme adragante, la gomme karaya, la gomme xanthane, les carraghénanes, les polymères pullulan ou agar, le chitosan ou ses dérivés, les carbomères, l'acide acrylique réticulé avec des polyalkényl éthers, les polycarbophiles, les copolymères de méthylvinyl et d'anhydride maléique, les poly-saccharides ou le mélange d'au moins deux de ces liants ; étant entendu que lorsque le liant comprend un ou plusieurs polysaccharides, le pourcentage en poids du ou desdits dit polysaccharides représente 50% ou moins, de préférence 25% ou moins, du poids total de liants ; ledit enrobage contenant entre 30% et 90% inclus en poids de matière appétissante par rapport au poids total de l'enrobage constitué de la matière appétissante et du liant ; ladite matière appétissante représentant au moins 5% et préférentiellement au moins 15% en poids de ladite composition appétissante pharmaceutique vétérinaire solide à administration orale.

14. Composition selon la revendication 13, **caractérisée en ce que** le principe actif de ladite composition pharmaceutique vétérinaire solide à administration orale est le pimobendan.

15. Utilisation de pimobendan pour la fabrication d'une composition appétissante pharmaceutique vétérinaire solide à administration orale pour la prévention et/ou le traitement de l'insuffisance cardiaque chez les animaux selon l'une quelconque des revendications 13 à 14.

## Patentansprüche

1. Verwendung einer Überzugszusammensetzung, die geeignet ist, durch ein Beschichtungsverfahren auf eine feste veterinärpharmazeutische Zusammensetzung zur oralen Verabreichung, welche wenigstens eine Verbindung umfasst, die in der Behandlung von Herzinsuffizienz verwendet wird, aufgetragen zu werden, wobei die pharmazeutische Zusammensetzung für die Prävention und/oder die Behandlung von Herzinsuffizienz bei Tieren indiziert ist, wobei die Überzugszusammensetzung **dadurch gekennzeichnet** ist, dass
(i) umfasst:
- ein Material, das für das Zieltier appetitanregend ist, das in Pulverform vorliegt und aus einer Substanz tierischer oder pflanzlicher Herkunft besteht, die direkt zu Pulver verarbeitet wurde;
- wenigstens ein Bindemittel, ausgewählt aus Polymeren von Polyvinylalkohol, Polyvinylpyrrolidon, Copolymeren von Vinylpyrrolidon und Vinylacetat, Polyvinylacetophthalat, Cellulosen und ihren Derivaten, Alginsäure und ihren Salzen, Zein, Hyaluronsäure, Pektinen, Gummi arabicum, Tragantgummi, Karayagummi, Xanthangummi, Carrageenanen, Pullulan- oder Agarpolymeren, Chitosan oder seinen Derivaten, Carbomeren, Acrylsäure, vernetzt mit Polyalkenylethern, Polycarbophilen, Copolymeren von Methylvinyl und Maleinsäureanhydrid, Polysacchariden oder einem Gemisch von wenigstens zwei dieser Bindemittel; mit der Maßgabe, dass, wenn das Bindemittel ein oder mehrere Polysaccharid(e) umfasst, der Gewichtsprozentanteil des Polysaccharids oder der Polysaccharide 50% oder weniger, vorzugsweise 25% oder weniger des Gesamtgewichts der Bindemittel darstellt, und
- ein Lösungsmittel, ausgewählt aus Wasser, Methanol, Ethanol, Isopropanol, Propylenglycol, Glycerin und deren Gemisch; und
(ii) sie zwischen 30 Gew.-% und 90 Gew.-%, einschließlich, appetitanregendes Material, bezogen auf das Gesamtgewicht des Gemisches, das aus dem Bindemittel und dem appetitanregenden Material besteht, enthält,
für den Überzug einer festen veterinärpharmazeutischen Zusammensetzung zur oralen Verabreichung durch Beschichtung.

2. Verwendung einer Überzugszusammensetzung gemäß Anspruch 1, **dadurch gekennzeichnet, dass** die feste veterinäre Zusammensetzung zur oralen Verabreichung, die für die Prävention und/oder die Behandlung von Herzinsuffizienz bei Tieren indiziert ist, auf der Basis von Pimobendan ist.

3. Verwendung einer Überzugszusammensetzung gemäß Anspruch 1 oder 2, **dadurch gekennzeichnet, dass** das pulverförmige appetitanregende Material aus Bierhefe, Fleischmehlen, Fischmehlen, Pulvern von Käsen oder Milchprodukten, Lebermehl und ihrem Gemisch ausgewählt ist.

4. Verwendung einer Überzugszusammensetzung gemäß einem der vorangehenden Ansprüche, **dadurch gekennzeichnet, dass** das appetitanregende pulverförmige Material aus Partikeln besteht, die einen mittleren Durchmesser von unter 500 µm, vorzugsweise unter 400 µm und äußerst bevorzugt von zwischen 50 und 100 µm haben.

5. Verwendung einer Überzugszusammensetzung gemäß einem der vorangehenden Ansprüche, **dadurch gekennzeichnet, dass** sie außerdem wenigstens ein Additiv umfasst, das aus Weichmachern, Stabilisatoren, Konservierungsmitteln, Füllstoffen, Surfactants, Färbemitteln und porenbildenden Mitteln ausgewählt ist.

6. Verwendung gemäß einem der vorangehenden Ansprüche, **dadurch gekennzeichnet, dass** die feste veterinärpharmazeutische Zusammensetzung zur oralen Verabreichung für ein fleischfressendes Haustier bestimmt ist.

7. Verfahren zur Herstellung einer festen veterinärpharmazeutischen Zusammensetzung zur oralen Verabreichung für die Prävention und/oder die Behandlung von Herzinsuffizienz bei Tieren, die wenigstens eine Verbindung umfasst, die in der Behandlung von Herzinsuffizienz verwendet wird, und die durch Beschichtung mit einem appetitanregenden Material überzogen ist, **dadurch gekennzeichnet, dass** es die folgenden Stufen umfasst:
(1) Auftragung auf eine feste veterinärpharmazeutische Zusammensetzung zur oralen Verabreichung für die Prävention und/oder die Behandlung von Herzinsuffizienz bei Tieren, die wenigstens eine Verbindung umfasst, die in der Behandlung von Herzinsuffizienz verwendet wird, einer Schicht aus einer Überzugszusammensetzung, umfassend:
- wenigstens ein Material, das für das Zieltier appetitanregend ist, das in Pulverform vorliegt und aus einer Substanz tierischer oder pflanzlicher Herkunft besteht, die direkt zu Pulver verarbeitet wurde;
- wenigstens ein Bindemittel, ausgewählt aus Polymeren von Polyvinylalkohol, Polyvinylpyrrolidon, Copolymeren von Vinylpyrrolidon und Vinylacetat, Polyvinylacetophthalat, Cellulosen und ihren Derivaten, Alginsäure und ihren Salzen, Zein, Hyaluronsäure, Pektinen, Gummi arabicum, Tragantgummi, Karayagummi, Xanthangummi, Carrageenanen, Pullulan- oder Agarpolymeren, Chitosan oder seinen Derivaten, Carbomeren, Acrylsäure, vernetzt mit Polyalkenylethern, Polycarbophilen, Copolymeren von Methylvinyl und Maleinsäureanhydrid, Polysacchariden oder einem Gemisch von wenigstens zwei dieser Bindemittel; mit der Maßgabe, dass, wenn das Bindemittel ein oder mehrere Polysaccharid(e) umfasst, der Gewichtsprozentanteil des Polysaccharids oder der Polysaccharide 50% oder weniger, vorzugsweise 25% oder weniger des Gesamtgewichts der Bindemittel darstellt, und
- ein Lösungsmittel, ausgewählt aus Wasser, Methanol, Ethanol, Isopropanol, Propylenglycol, Glycerin und deren Gemisch;
wobei die Überzugszusammensetzung zwischen 30 Gew.-% und 90 Gew.-%, einschließlich, appetitanregendes Material, bezogen auf das Gesamtgewicht des Gemisches, das aus dem Bindemittel und dem appetitanregenden Material besteht, enthält;
(2) Trocknung der überzogenen Zusammensetzung, die in Stufe (1) erhalten wurde;
(3) gegebenenfalls Wiederholung der Stufen (1) und (2) und
(4) Erhalt einer festen veterinärpharmazeutischen appetitanregenden Zusammensetzung zur oralen Verabreichung für die Prävention und/oder die Behandlung von Herzinsuffizienz bei Tieren, in der das appetitanregende Material wenigstens 5 Gew.-% und vorzugsweise wenigstens 15 Gew.-% der festen veterinärpharmazeutischen appetitanregenden Zusammensetzung zur oralen Verabreichung darstellt.

8. Verfahren gemäß Anspruch 7, **dadurch gekennzeichnet, dass** die feste veterinäre Zusammensetzung zur oralen Verabreichung für die Prävention und/oder die Behandlung von Herzinsuffizienz bei Tieren Pimobendan umfasst.

9. Verfahren gemäß einem der Ansprüche 7 oder 8, **dadurch gekennzeichnet, dass** die feste veterinärpharmazeutische Zusammensetzung zur oralen Verabreichung eine gepresste Tablette, eine harte Gelatinekapsel, eine weiche Kapsel, eine Pille, eine Tablette, ein Granulat, eine Tablette (Cachet), ein Kaugummi oder eine Pastille ist.

10. Verfahren gemäß Anspruch 9, **dadurch gekennzeichnet, dass** die feste veterinärpharmazeutische Zusammensetzung zur oralen Verabreichung für die Prävention und/oder die Behandlung von Herzinsuffizienz bei Tieren eine harte Gelatinekapsel ist.

11. Verfahren gemäß einem der Ansprüche 7 bis 9, **dadurch gekennzeichnet, dass** die feste veterinärpharmazeutische Zusammensetzung zur oralen Verabreichung für die Prävention und/oder die Behandlung von Herzinsuffizienz bei Tieren teilbar ist.

12. Verfahren gemäß einem der Ansprüche 7 bis 11, **dadurch gekennzeichnet, dass** es eine zusätzliche Stufe vor Stufe (1) umfasst, die darin besteht, die feste veterinärpharmazeutische Zusammensetzung zur oralen Verabreichung mit einem primären Film mittels einer Lösung oder Suspension, die wenigstens ein Bindemittel und wenigstens ein Lösungsmittel und/oder ein annehmbares Additiv oder annehmbare Additive umfasst, zu überziehen.

13. Feste veterinärpharmazeutische appetitanregende Zusammensetzung zur oralen Verabreichung für die Prävention und/oder die Behandlung von Herzinsuffizienz bei Tieren, **dadurch gekennzeichnet, dass** sie umfasst:
- eine feste veterinärpharmazeutische Zusammensetzung zur oralen Verabreichung, die wenigstens einen Wirkstoff enthält, der aus einer Verbindung besteht, die in der Behandlung von Herzinsuffizienz verwendet wird, und
- einen Überzug, aufgetragen durch Beschichtung, angeordnet um die Zusammensetzung, bestehend aus einem appetitanregenden Material, das sich in Pulverform präsentiert, bestehend aus einer Substanz tierischer oder pflanzlicher Herkunft, die direkt zu Pulver verarbeitet wurde, und wenigstens einem Bindemittel, ausgewählt aus Polymeren von Polyvinylalkohol, Polyvinylpyrrolidon, Copolymeren von Vinylpyrrolidon und Vinylacetat, Polyvinylacetophtalat, Cellulosen und ihren Derivaten, Alginsäure und ihren Salzen, Zein, Hyaluronsäure, Pectinen, Gummi arabicum, Tragantgummi, Karayagummi, Xanthangummi, Carrageenanen, Pullulan- oder Agarpolymeren, Chitosan und seinen Derivaten, Carbomeren, Acrylsäure, vernetzt mit Polyalkenylethern, Polycarbophilen, Copolymeren von Methylvinyl und Maleinsäureanhydrid, Polysacchariden und einem Gemisch von wenigstens zwei dieser Bindemittel; mit der Maßgabe, dass, wenn das Bindemittel ein Polysaccharid oder mehrere Polysaccharide umfasst, der Gewichtsprozentanteil des Polysaccharids oder der Polysaccharide 50% oder weniger, vorzugsweise 25% oder weniger des Gesamtgewichts der Bindemittel darstellt; wobei der Überzug zwischen 30 Gew.-% und 90 Gew.-%, einschließlich appetitanregendem Material, bezogen auf das Gesamtgewichts des Überzugs, das aus dem appetitanregenden Material und dem Bindemittel besteht, enthält; wobei das appetitanregende Material wenigstens 5 Gew.-% und vorzugsweise wenigstens 15 Gew.-% der festen veterinärpharmazeutischen appetitanregenden Zusammensetzung zur oralen Verabreichung darstellt.

14. Zusammensetzung gemäß Anspruch 13, **dadurch gekennzeichnet, dass** der Wirkstoff der festen veterinärpharmazeutischen Zusammensetzung zur oralen Verabreichung Pimobendan ist.

15. Verwendung von Pimobendan für die Herstellung einer festen veterinärpharmazeutischen appetitanregenden Zusammensetzung zur oralen Verabreichung für die Prävention und/oder die Behandlung von Herzinsuffizienz bei Tieren gemäß einem der Ansprüche 13 bis 14.

## Claims

1. The use of a coating composition capable of being applied by a film-coating process to a solid veterinary pharmaceutical composition for oral administration comprising at least one compound used for the treatment of cardiac insufficiency, said pharmaceutical composition being indicated for the prevention and/or treatment of cardiac insufficiency in animals, **characterized in that**:
(i) it comprises:
- a material appetizing for the target animal, which is provided in the pulverulent form and which is chosen from substances of animal or plant origin, directly brought to a powder after treatment;
- at least one binder chosen from polyvinyl alcohol polymers, polyvinylpyrrolidone polymers, copolymers of vinylpyrrolidone and of vinyl acetate, polyvinyl acetate phthalate, celluloses and their derivatives, alginic acid and its salts, zein, hyaluronic acid, pectins, gum arabic, gum tragacanth, karaya gum, xanthan gum, carrageenans, pullulan or agar polymers, chitosan or its derivatives, carbomers, acrylic acid crosslinked with polyalkenyl ethers, polycarbophils, copolymers of methyl vinyl and of maleic anhydride, polysaccharides or the mixture of at least two of these binders; it being understood that, when the binder comprises one or more polysaccharides, the percentage by weight of said polysaccharides represents 50% or less, preferably 25% or less, of the total weight of binders; and
- a solvent chosen from water, methanol, ethanol, isopropanol, propylene glycol, glycerol or their mixture;
and (ii) it comprises between 30 and 90% inclusive by weight of appetizing material, with respect to the total weight of the mixture composed of the binder and the appetizing material,
in the coating by film-coating of a solid veterinary pharmaceutical composition for oral administration.

2. The use of a coating composition as claimed in claim 1, **characterized in that** said solid veterinary composition for oral administration indicated for the prevention and/or treatment of cardiac insufficiency in animals is based on pimobendan.

3. The use of a coating composition as claimed in claim 1 or 2, **characterized in that** said pulverulent appetizing material is chosen from beer yeast, meat meals, fish meals, powdered cheeses or milk derivatives, liver powder and their mixture.

4. The use of a coating composition as claimed in any one of the preceding claims, **characterized in that** said pulverulent appetizing material is composed of particles having a mean diameter of less than 500 µm, preferably of less than 400 µm and more preferably of between 50 and 100 µm.

5. The use of a coating composition as claimed in any one of the preceding claims, **characterized in that** it additionally comprises at least one additive chosen from plasticizers; stabilizing agents; preservatives; fillers; surfactants; colorants and porogenic agents.

6. The use as claimed in any one of the preceding claims, **characterized in that** said solid veterinary pharmaceutical composition for oral administration is intended for a domestic carnivorous animal.

7. A process for the preparation of a solid veterinary pharmaceutical composition for oral administration for the prevention and/or treatment of cardiac insufficiency in animals comprising at least one compound used for the treatment of cardiac insufficiency, said composition being coated by film-coating with an appetizing material, **characterized in that** it comprises the following stages:
(1) application, to a solid veterinary pharmaceutical composition for oral administration for the prevention and/or treatment of cardiac insufficiency in animals comprising at least one compound used for the treatment of cardiac insufficiency, of a layer of a coating composition comprising
- at least one appetizing material for the target animal in the pulverulent form and chosen from substances of animal or plant origin, directly brought to a powder after treatment;
- at least one binder chosen from polyvinyl alcohol polymers, polyvinylpyrrolidone polymers, copolymers of vinylpyrrolidone and of vinyl acetate, polyvinyl acetate phthalate, celluloses and their derivatives, alginic acid and its salts, zein, hyaluronic acid, pectins, gum arabic, gum tragacanth, karaya gum, xanthan gum, carrageenans, pullulan or agar polymers, chitosan or its derivatives, carbomers, acrylic acid crosslinked with polyalkenyl ethers, polycarbophils, copolymers of methyl vinyl and of maleic anhydride, polysaccharides or the mixture of at least two of these binders; it being understood that, when the binder comprises one or more polysaccharides, the percentage by weight of said polysaccharides represents 50% or less, preferably 25% or less, of the total weight of binders; and
- a solvent chosen from water, methanol, ethanol, isopropanol, propylene glycol, glycerol or their mixture;
said coating composition comprises between 30 and
90% inclusive by weight of appetizing material, with respect to the total weight of the mixture composed of the binder and the appetizing material;
(2) drying said coated composition obtained in stage (1);
(3) optionally, repetition of stages (1) and (2); and
(4) production of a solid veterinary pharmaceutical appetizing composition for oral administration for the prevention and/or treatment of cardiac insufficiency in animals in which said appetizing material represents at least 5% by weight and preferably at least 15% by weight of said solid veterinary pharmaceutical appetizing composition for oral administration.

8. The process as claimed in claim 7, **characterized in that** said solid veterinary composition for oral administration for the prevention and/or treatment of cardiac insufficiency in animals comprises pimobendan.

9. The process as claimed in either one of claims 7 and 8, **characterized in that** said solid veterinary pharmaceutical composition for oral administration is a tablet, including a compressed tablet, a hard gelatin capsule, a soft capsule, a pill, a lozenge, granules, a chewing gum or a pastille.

10. The process as claimed in claim 9, **characterized in that** said solid veterinary pharmaceutical composition for oral administration for the prevention and/or treatment of cardiac insufficiency in animals is a hard gelatin capsule.

11. The process as claimed in any one of claims 7 to 9, **characterized in that** said solid veterinary pharmaceutical composition for oral administration for the prevention and/or treatment of cardiac insufficiency in animals is scored.

12. The process as claimed in any one of claims 7 to 11, **characterized in that** it comprises an additional stage prior to stage (1) which consists in coating said solid veterinary pharmaceutical composition for oral administration with a primer film using a solution or suspension comprising at least one binder and at least one solvent and/or one or more acceptable additives.

13. A solid veterinary pharmaceutical appetizing composition for oral administration for the prevention and/or treatment of cardiac insufficiency in animals, **characterized in that** it comprises:
- a solid veterinary pharmaceutical composition for oral administration comprising at least one active principle consisting in a compound used for the treatment of cardiac insufficiency; and
- a coating applied by film-coating and positioned around said composition composed of an appetizing material in the pulverulent form and chosen from substances of animal or plant origin, directly brought to a powder after treatment and of at least one binder chosen from polyvinyl alcohol polymers, polyvinylpyrrolidone polymers, copolymers of vinylpyrrolidone and of vinyl acetate, polyvinyl acetate phthalate, celluloses and their derivatives, alginic acid and its salts, zein, hyaluronic acid, pectins, gum arabic, gum tragacanth, karaya gum, xanthan gum, carrageenans, pullulan or agar polymers, chitosan or its derivatives, carbomers, acrylic acid crosslinked with polyalkenyl ethers, polycarbophils, copolymers of methyl vinyl and of maleic anhydride, polysaccharides or the mixture of at least two of these binders; it being understood that, when the binder comprises one or more polysaccharides, the percentage by weight of said polysaccharides represents 50% or less, preferably 25% or less, of the total weight of binders; said coating comprising between 30 and 90% inclusive by weight of appetizing material, with respect to the total weight of the coating composed of the appetizing material and of the binder, said appetizing material representing at least 5% by weight and preferably at least 15% by weight of said solid veterinary pharmaceutical appetizing composition for oral administration.

14. The composition as claimed in claim 13, **characterized in that** the active principle of said solid veterinary pharmaceutical composition for oral administration is pimobendan.

15. The use of pimobendan in the manufacture of a solid veterinary pharmaceutical appetizing composition for oral administration for the prevention and/or treatment of cardiac insufficiency in animals as claimed in any one of claims 13 to 14.
